# EUROPEAN PATENT SPECIFICATION

(11) **EP 3 461 490 B1**
(45) Date of publication and mention of the grant of the patent: **13.03.2024**
(21) Application number: 18197760.4
(22) Date of filing: 28.09.2018
(51) Int. Cl.: A61K 38/08, A61K 36/77, A61K 38/39, A61P 21/02

(54) **PHARMACEUTICAL COMPOSITION FOR TREATING HEMORRHOIDAL DISAESE**
PHARMAZEUTISCHE ZUSAMMENSETZUNG ZUR BEHANDLUNG VON HÄMORRHOIDALEN ERKRANKUNGEN
COMPOSITION PHARMACEUTIQUE POUR LE TRAITEMENT DE LA MALADIE HÉMORROÏDAIRE

(30) Priority: 29.09.2017 IT 201700109691
(43) Date of publication of application: 03.04.2019
(73) Proprietor: Uniderm Farmaceutici S.r.l., 00125 Rome (IT)
(72) Inventor: Olmo Sara, 00124 Roma (RM) (IT); Sabato Anna, 00060 Capena (RM) (IT); Chiozza Giorgio, 00125 Roma (RM) (IT); Migliuolo Moira, 00125 Roma (RM) (IT)
(74) Representative: Primiceri, Maria Vittoria

(56) References cited:
- EP-A1- 3 087 993
- DATABASE GNPD [Online] MINTEL; 5 April 2016 (2016-04-05), anonymous: "Targeted Filler with A-F33", XP055533126, retrieved from www.gnpd.com Database accession no. 3904793
- DATABASE GNPD [Online] MINTEL; 11 May 2017 (2017-05-11), anonymous: "Restoring Night Eye Gels", XP055533127, retrieved from www.gnpd.com Database accession no. 4713335
- DATABASE GNPD [Online] MINTEL; 7 October 2014 (2014-10-07), anonymous: "Wrinkle Spot Treatment", XP055533135, retrieved from www.gnpd.com Database accession no. 2707767
- Anonymous: "anonet-plus-crema-emolliente", , 1 January 2016 (2016-01-01), XP055474903, Retrieved from the Internet: URL:https://www.efarma.com/anonet-plus-cre ma-emolliente-30-grammi.html [retrieved on 2018-05-14]

## Description

### Technical field of the invention

This invention relates to pharmaceutical compositions for use in the treatment of haemorrhoids.

### Known art

Haemorrhoids are cushions of vascular tissue arising from abnormal dilation of the veins located in the anal canal and the terminal part of the rectum.

Haemorrhoid cushions have an important part to play in the maintenance of faecal continence; in addition to this haemorrhoids act to protect the anal sphincter during the passage of faeces.

Normally the presence of these vascular cushions is not noticed, but in particular circumstances haemorrhoids can swell, giving rise to the symptoms of haemorrhoids.

By the term "haemorrhoids" is meant a particular pathological condition in which the haemorrhoid vessels dilate excessively to the point of collapse and congestion, that is weakening and losing elasticity. The term "piles" is commonly used to describe haemorrhoids.

The term haemorrhoids is used when the cushions of haemorrhoid tissue increase in volume and prolapse together with the rectal mucosa, that is they protrude from the anal canal, when they become the location of congestion and bruising and begin to bleed, and when a pruriginous and painful blood clot (thrombus) forms within them.

Haemorrhoids are initially classified on the basis of the location where they occur.
- Internal haemorrhoids. These are located within the rectum and in general do not cause any particular discomfort. However, straining during evacuation can damage the surface causing bleeding, and cause the haemorrhoid cushions to slide out (projecting haemorrhoids or haemorrhoidal prolapse), causing irritation and pain.
- External haemorrhoids. These are found under the skin surrounding the anus. When they are irritated they can cause itching or bleeding. Coagulated blood (thrombus) may also form, with consequent swelling, inflammation and pain.

External haemorrhoids show the strongest symptoms. If they form thrombi they can swell and become very painful. Internal ones on the other hand are often asymptomatic and if characterised by the presence of thrombi can give rise to bleeding.

In medicine haemorrhoids are therefore classified into four grades or stages depending upon the severity of the clinical picture:
- 1^{st} grade. There is an increase in the volume of one or more haemorrhoidal cushions with discomfort, pruritus and possible bleeding during defecation.
- 2^{nd} grade. Initial haemorrhoidal prolapse occurs (the haemorrhoids protrude from the anal canal), only during defecation, with subsequent spontaneous reduction. Discomfort, pruritus and bleeding may occur.
- 3^{rd} grade. The haemorrhoidal prolapse requires manual reduction. Symptoms include discomfort, pruritus, bleeding, pain and mild faecal incontinence.
- 4^{th} grade. The prolapse is permanent, not manually reducible. The symptoms are pain, intense itching, and constant faecal incontinence.

The therapeutic approach to haemorrhoids comprises actions of various kinds depending upon severity and symptoms, from a mere change in lifestyle, to the prescription of medical treatment, and ultimately to the application of mini-invasive or fully surgical techniques when the stage of the disease or failure of the symptoms to regress requires assistance.

Surgery is however only recommended in cases of severe haemorrhoids or haemorrhoids which are resistant to dietary measures and pharmacological treatments.

The inventors are aware of various pharmaceutical compositions for the topical treatment of haemorrhoids produced in the form of ointments and suppositories.

Haemorrhoid treatments may for example be based on cortisones (hydrocortisone, fluocortolone, dexamethasone, fluocinolone), contact anaesthetics (tetracaine, oxetacaine, pramocaine), and substances having an anti-inflammatory, vasoconstrictive and spasmolytic action in the vicinity of the anal sphincter.

From the pharmacological point of view, pharmaceutical compositions based on cortisone molecules are considered to be extremely efficacious; these compositions are commonly used to cure many irritations, but have not a few side effects. Cortisone drugs can in fact cause skin irritation and sensitisation problems to occur and cannot be used for long periods of time, or when scarring, broken skin or infections are present.

Pharmaceutical compositions for topical use based on molecules having an anaesthetic function are also available for the treatment of haemorrhoids; in general contact anaesthetics (tetracaine, oxetacaine, pramocaine) which alleviate pain and itching in the anal area are used. The use of such compositions should however be limited over time, particularly in the case where the anal mucosa is damaged.

If used for long periods all these preparations can give rise to irritation or skin allergies which manifest themselves as inflammation of the follicles, burning, itching or dryness of the skin.

There are other diseases of the anal and perianal region, such as for example fissures, which are often erroneously confused with haemorrhoids. Haemorrhoids and fissures are in fact both disturbances which can affect the anal and perianal area, but which are due to different causes. The term "haemorrhoids" or piles is therefore a disease which affects vascular structures belonging to the anal canal. The most commonly used topical treatments for conditions of this type are products which mainly act on the microcirculation, often also accompanied by an anti-inflammatory action. "Anal fissures" are instead a linear ulceration of the anus, sometimes alone and solitary, mainly located along the posterior median line. Of a few millimetres in length, they are located along the boundary between the pectinate line and the anoderm. Their presence causes painful spasms of the anal sphincter. Elective topical treatments for anal fissures are intended to act on anal hypertonia (nifedipine, nitroglycerin, trinitroglycerin, botulinus toxin) in addition to acting on pain (lidocaine). The pharmacological approaches currently available for the treatment of fissures are not useful for the treatment of haemorrhoids because the topical preparations used for the treatment of this disease do not provide for the presence of substances working on the changed microcirculation, the primary elective cause of haemorrhoids.

There has therefore been felt to be a need for a safe and noninvasive topical approach which can be used over long term without side effects to reduce the clinical symptoms of local inflammation and improve trophism of the haemorrhoidal vascular tissue.

Argireline^{®}, also known as Acetyl Hexapeptide-8 (Ac-Glu-Glu-Met-Gln-Arg-Arg-NH2 -SEQ ID NO:1) is a peptide used to treat anal fissures (EP 3087993).

Leuphasyl^{®}, also known as Pentapeptide-18 (H-Tyr-D-Ala-Gly-Phe-Leu-OH, SEQ ID NO:2), is another peptide used to treat anal fissures (EP 3087993).

The following documents: DATABASE GNPD [Online] MINTEL; 5 April 2016 (2016-04-05), anonymous: "Targeted Filler with A-F33", XP055533126, retrieved from www.gnpd.com Database accession no. 3904793; DATABASE GNPD [Online] MINTEL; 11 May 2017 (2017-05-11), anonymous: "Restoring Night Eye Gels", XP055533127, retrieved from www.gnpd.com Database accession no. 4713335; DATABASE GNPD [Online] MINTEL; 7 October 2014 (2014-10-07), anonymous: "Wrinkle Spot Treatment", XP055533135, retrieved from www.gnpd.com Database accession no. 2707767; disclose cosmetic products containing Argireline and/or Leuphasys in combination with *Aesculus Hippocastanum extract, Centella extract* and *Hamamelis extract.*

Document Anonymous: "anonet-plus-crema-emolliente", 1 January 2016 (2016-01-01), XP055474903, retrieved from the Internet: URL:https://www.efarma.com/anonet-plus-crema-emolliente-30-grammi.html describes a composition for treating hemorrhoids comprising *Aesculus Hippocastanum glycolic extract, Centella glycolic extract* and *Hamamelis glycolic extract.*

### Summary of the invention

An unexpected effect on haemorrhoids from Acetyl Hexapeptide-8 and Pentapeptide-18 alone or in combination has now been found; this unexpected effect also increases synergistically when the peptides are used in combination with at least one plant extract having activity on the microcirculation preferably selected from the group Hippocastanum, Centella, Ilex, Vaccinium, Hamamelis, Vitis, Melilotus, Holly, Cypress, Achillea, Ginkgo biloba, Boraginaceae, Urtica, Orthosiphon, Sambucus, Citrus and mixtures thereof.

The combination of Acetyl Hexapeptide-8 with Pentapeptide-18 and the extracts according to the invention has demonstrated an unexpected effect on reducing clinical symptoms and local inflammation and improving trophism of the haemorrhoidal vascular tissue.

The object of the present invention is therefore a pharmaceutical composition comprising Acetyl Hexapeptide-8 and Pentapeptide-18 in combination with at least one plant extract having activity on the microcirculation, preferably selected from the group Hippocastanum, Centella, Ilex, Vaccinium, Hamamelis, Vitis, Melilotus, Holly, Cypress, Achillea, Ginkgo biloba, Boraginaceae, Urtica, Orthosiphon, Sambucus, Citrus and mixtures thereof.

An object of the present invention is a pharmaceutical composition comprising Acetyl Hexapeptide-8 and Pentapeptide-18 in combination with a hippocastanum extract.

Yet another object of the present invention is a pharmaceutical composition comprising Acetyl Hexapeptide-8 and Pentapeptide-18 in combination with at least one plant extract having activity on the microcirculation preferably selected from the group Hippocastanum, Centella, Ilex, Vaccinium, Hamamelis, Vitis, Melilotus, Holly, Cypress, Achillea, Ginkgo biloba, Boraginaceae, Urtica, Orthosiphon, Sambucus, Citrus and mixtures thereof for use in the medical field, and in particular for use in the treatment of haemorrhoids and to improve trophism of the haemorrhoidal vascular tissue.

Another object of the invention is a kit comprising the pharmaceutical composition comprising Acetyl Hexapeptide-8 and Pentapeptide-18 in combination with at least one plant extract according to the invention and a reusable or disposable applicator containing effective doses of the composition.

Further aspects will be apparent from the detailed description of the invention.

### Description of the figures

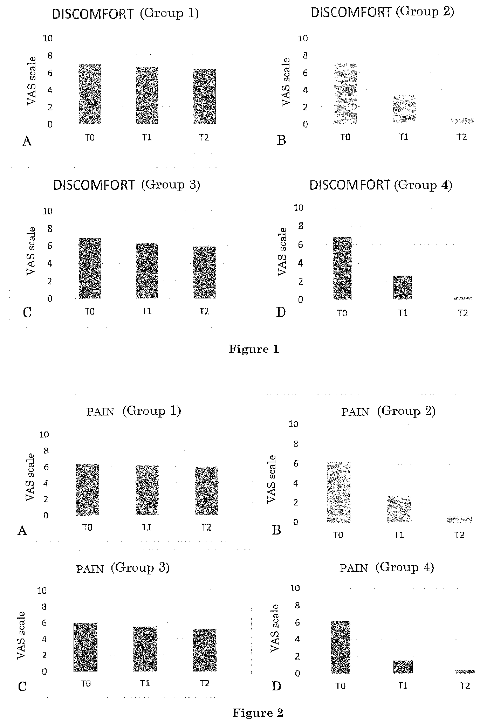

**Figure 1****:** Graphs showing the results of statistical analysis of data obtained from measuring discomfort on the VAS (Visual Analog Scale) made at time T0 (time of enrolment), Time T1 (after 7 days treatment) and Time T2 (after 30 days treatment). The various panels show the data relating to patients in the different treatment groups: group 1 (panel A), group 2 (panel B), group 3 (panel C) and group 4 (panel D).
**Figure 2****:** Graphs showing the results of statistical analysis of data obtained from measuring pain on the VAS (Visual Analog Scale) made at time T0 (time of enrolment), Time T1 (after 7 days treatment) and Time T2 (after 30 days treatment). The various panels show the data relating to patients in the different treatment groups: group 1 (panel A), group 2 (panel B), group 3 (panel C) and group 4 (panel D).

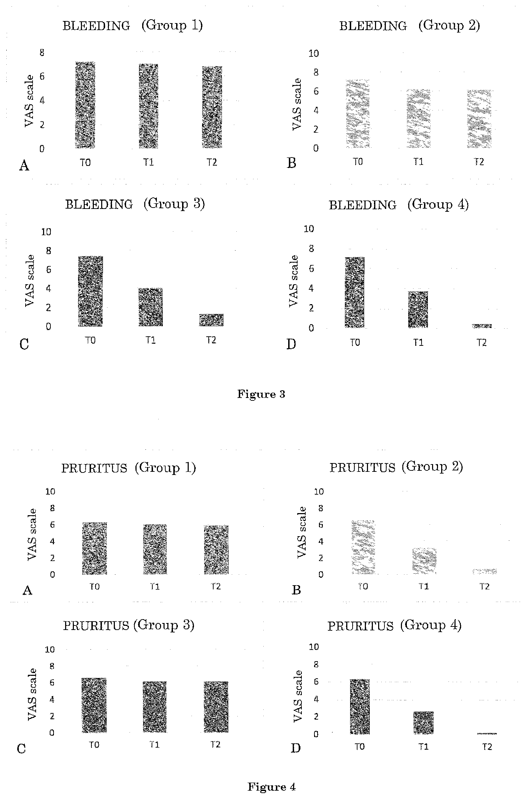

**Figure 3****:** Graphs showing the results of statistical analysis of data obtained from measuring bleeding on the VAS (Visual Analog Scale) made at time T0 (time of enrolment), Time T1 (after 7 days treatment) and Time T2 (after 30 days treatment). The various panels show the data relating to patients in the different treatment groups: group 1 (panel A), group 2 (panel B), group 3 (panel C) and group 4 (panel D).
**Figure 4****:** Graphs showing the results of statistical analysis of data obtained from measuring pruritus on the VAS (Visual Analog Scale) made at time T0 (time of enrolment), Time T1 (after 7 days treatment) and Time T2 (after 30 days treatment). The various panels show the data relating to patients in the different treatment groups: group 1 (panel A), group 2 (panel B), group 3 (panel C) and group 4 (panel D).

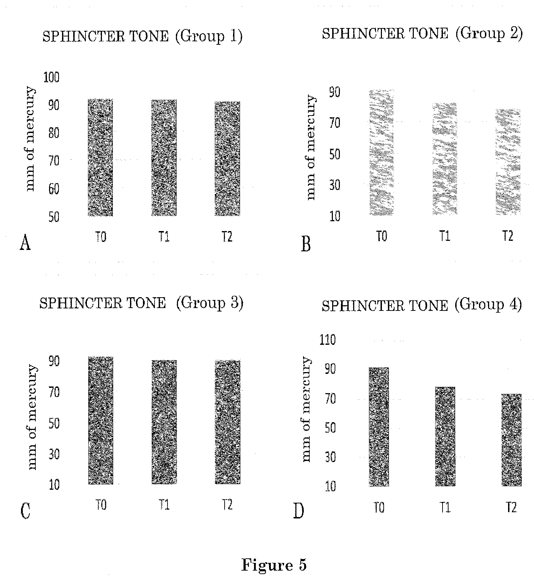

**Figure 5****:** Graphs showing the results of statistical analysis of data obtained from measuring sphincter tone made at time T0 (time of enrolment), Time T1 (after 7 days treatment) and Time T2 (after 30 days treatment). The various panels show the data relating to patients in the different treatment groups: group 1 (panel A), group 2 (panel B), group 3 (panel C) and group 4 (panel D).

### Detailed description of the invention

The inventors have now found a surprising improving effect on the symptoms of haemorrhoids provided by Acetyl Hexapeptide-8 and Pentapeptide-18 alone or in combination; a composition containing at least one of these two peptides is capable of bringing about an unexpected improvement in both painful conditions of the anal region associated with the symptoms of haemorrhoids, and trophism of the haemorrhoidal vascular tissue.

The present invention therefore relates to a pharmaceutical composition comprising Acetyl Hexapeptide-8 or Pentapeptide-18 alone or in combination for use in the treatment of haemorrhoids, in particular in the treatment of the painful symptoms caused by local inflammation and to improve trophism of the haemorrhoidal vascular tissue.

The inventors have also unexpectedly found that there is a synergistic effect if the mixture of Pentapeptide-18 and Acetyl Hexapeptide-8 is used in combination with at least one plant extract having activity on the microcirculation, and in particular extracts of hippocastanum, Centella, Ilex, Vaccinium, Hamamelis, Vitis, Melilotus, Holly, Cypress, Achillea, Ginkgo biloba, Boraginaceae, Urtica, Orthosiphon, Sambucus, Citrus and their corresponding mixtures have proved to be particularly effective. These extracts have been selected on the basis their effective anti-inflammatory action and their tropic activity on the microcirculation. According to the invention it is also possible to use other plant extracts comprising substances having activity on the microcirculation; the compositions so formed are in fact capable of producing even more obvious effects than those to be expected from a mere combination of the improving effects due to the molecule selected or the mixture of Pentapeptide-18 and Acetyl Hexapeptide-8.

The plant extracts mentioned above are in fact characterised by the presence of compounds which through acting on the microcirculation can be regarded as having potentially antihaemorrhoidal activity.

The preferred extract according to the invention is hippocastanum extract.

The present invention therefore relates to a pharmaceutical composition comprising Acetyl Hexapeptide-8 and Pentapeptide-18 in combination with at least one plant extract having activity on the microcirculation preferably selected from the group Hippocastanum, Centella, Ilex, Vaccinium, Hamamelis, Vitis, Melilotus, Holly, Cypress, Achillea, Ginkgo biloba, Boraginaceae, Urtica, Orthosiphon, Sambucus, Citrus and corresponding mixtures and use of such composition in the medical field and in particular in the treatment of haemorrhoids, in particular in the treatment of the painful symptoms caused by local inflammation and to improve trophism of the haemorrhoidal vascular tissue.

The pharmaceutical compositions according to the invention are preferably used in the treatment of grade I and II haemorrhoids or in the treatment of III and IV grade haemorrhoids during the pre- and/or post-surgery stage.

All the percentages shown below are to be regarded as percentages by weight.

In the context of the present invention Acetyl Hexapeptide-8 is used in concentrations from 0.00005% to 0.01%, preferably between 0.003% and 0.007%.

In a preferred embodiment Acetyl Hexapeptide-8 is the commercial product Argireline^{®}.

Pentapeptide-18 is used in concentrations from 0.000025% to 0.005%, preferably between 0.001% and 0.004%.

In a preferred embodiment the Pentapeptide-18 is the commercial product Leuphasyl^{®}.

In a preferred embodiment a mixture of Pentapeptide-18 and Acetyl Hexapeptide-8 is the commercial product Argirelox^{®} and in a preferred embodiment this mixture is used in concentrations from 0.1 to 20%, preferably between 8% and 12%.

The extracts according to the invention may be prepared according to the methods conventionally used in the field of the extraction of natural products known to those skilled in the art or as for example described in *"Botanicals, a Phytocosmetic Desk Reference"* by Frank S. D'Amelio, CRC Press, pages 39-48.

In the context of the present invention plant extracts of Hippocastanum, Centella, Ilex, Vaccinium, Hamamelis, Vitis, Melilotus, Holly, Cypress, Achillea, Ginkgo biloba, Boraginaceae, Urtica, Orthosiphon, Sambucus, Citrus or any other plant extract comprising substances having activity on the microcirculation are preferred.

In the context of the present invention the plant extract from hippocastanum and more particularly a glycolic mixture of hippocastanum is particularly preferred.

A glycolic mixture of hippocastanum or glycolic hippocastanum extract is used according to the invention in concentrations from 0.01% to 10%, preferably between 1% and 5%.

The hippocastanum active ingredient "AESCULUS HIPPOCASTANUM EXTRACT", present in the commercial product named "Glycolic hippocastanum extract" can be used in concentrations varying from 0.01% to 10%, preferably between 0.05% and 2%.

The composition according to the invention has a pH of between 3.5 and 6.5, preferably between 4.5 and 5.5.

According to the invention the composition may contain one or more pharmaceutically acceptable additives and excipients such as preservatives, antioxidants, acidifiers, chelating agents, perfumes, rheological additives, solubilising agents, neutralising agents.

In particular Imidazolidinyl Urea, benzoic acid or its salts or esters, sorbic acid or its salts, 4-hydroxybenzoic acid or its salts or esters, phenoxyethanol, dimethylol dimethyl hydantoin (DMDM Hydantoin), benzyl alcohol, isothiazolinones, dehydroacetic acid and its sodium salt, parabens, vitamins (groups A, C, E), lecithin, arginine, soda, citric acid, lactic acid, carbomer and its derivatives, hydroxyethylcellulose, xanthan gum, acrylate polymers and EDTA can advantageously be used.

The preservatives are preferably Imidazolidinyl Urea, potassium sorbate and phenoxyethanol.

The particularly preferred acidifier is citric acid.

According to the invention the concentration of Imidazolidinyl Urea may vary between 0.1% and 1%, preferably between 0.25% and 0.40%.

According to the invention the concentration of citric acid may vary between 0.1% and 1%, preferably between 0.20% and 0.40%,

According to the invention the potassium sorbate concentration may vary between 0.1% and 1%, preferably between 0.25% and 0.60%.

According to the invention the concentration of phenoxyethanol may vary between 0.1% and 1%, preferably between 0.3% and 0.80%.

According to the invention the composition may contain one or more humidifiers, hydrating agents, mollifying agents, surfactants and emulsifiers selected from glycerine, butylene glycol, pentylene glycol, hydroxyphenyl propamidobenzoic acid, 4-t-butylcyclohexanol, polyoxyethylene stearate, glyceryl stearate, dimethicone, phenyl-trimethicone, pantenol, synthetic or natural triglycerides, fatty acid esters, glycol-stearate or its esters, cetylstearyl alcohol, pantenol, plant extracts, bisabolol, aloe vera, plant oils, elastin and collagen.

The glycerine concentration may vary between 1% and 10%, preferably between 3% and 8%.

The concentration of triglycerides, preferably C10-18 triglycerides, may vary between 5% and 20%, preferably between 12% and 18%.

The concentration of C12-20 acid/PEG-8 ester may vary between 2% and 20%, preferably between 5% and 10%.

The concentration of glycol-stearate acetylate may vary between 1% and 10%, preferably between 2% and 5%.

The concentration of cetylstearyl alcohol may vary between 0.5% and 10%, preferably between 1% and 3%.

The concentration of hydroxyphenyl-propamidobenzoic acid may vary between 0.005% and 0.2%, preferably between 0.03% and 0.07%.

The concentration of glyceryl-stearate may vary between 0.1% and 5%, preferably between 0.5% and 1.8%.

The concentration of the mixture of polyoxyethylene (10) isocetylstearate and polyoxyethylene (10) isostearyl-stearate may vary between 0.1% and 2%, preferably between 0.15% and 1%.

The concentration of dimethicone may vary between 0.1% and 10%, preferably between 0.3% and 3%.

The concentration of the glyceryl-stearate and PEG-100 stearate mixture may vary between 0.5% and 10%, preferably between 0.5% and 3%.

In the context of the present invention the 4-t-butylcyclohexanol is in concentrations which vary between 0.01% and 3%, preferably between 0.2% and 1.0%.

In a preferred embodiment the 4-t-butylcyclohexanol is the commercial product Symsitive and this product is used in concentrations from 0.01% to 10%, preferably between 0.5% and 4%.

In a preferred embodiment a mixture of hydroxyphenyl-propamidobenzoic acid, 4-t-butylcyclohexanol, butylene-glycol and pentylene-glycol is the commercial product Symcalmin^{®} and this product is used in concentrations from 0.05% to 5%.

According to the invention the composition may also contain one or more elasticising or cicatrising agents such as Dermonectin^{®} or Dermonectin (Vevy, code 18.1926), also known as oligopeptide-5, which is an active peptide widely used in the field of cosmetics (Lexicon Vevy Skin Care Instant Reports Year I, No. 1, June 1984). It is characterised by the tetrapeptide having the L-arginyl-glycyl-L-aspartyl-L-serine sequence (SEQ ID NO: 3). According to the invention Dermonectin is used in a concentration varying between 0.01% and 10%, and preferably between 2% and 3%, or oligopeptide-5 in a concentration varying from 0.004% and 4% and preferably between 0.8% and 2.5%.

The active ingredient 4-t-butylcyclohexanol present in the commercial product Dermonectin^{®} may be used in concentrations varying from 0.004 to 4%, preferably between 0.25% and 2%.

The solvent used to bring the final weight of the composition up to 100% is selected from water, alcohols, glycols and oils selected from those normally used in the cosmetic and pharmaceutical fields.

According to one preferred embodiment the composition is made in the form of a gel, cream, emulsion, ointment, stick, spray, enema or suppository or in any other form suitable for topical administration known to those skilled in the art.

According to the invention the composition may contain at least one pharmaceutically acceptable vehicle or excipient.

The composition according to the invention may be made in such a way as to permit release of the various active ingredients to be simultaneous, sequential or delayed.

According to the invention the composition may be used in combination with other active ingredients known to be effective such as muscular relaxants for simultaneous, sequential or delayed administration.

Preparation of the composition in the form of a cream for example takes place according to a method characterised by the following steps:
The starting materials are placed in a blender and turbo emulsifier in the manner and quantities specified by the formula.

The starting materials placed in the blender melt at a temperature which can vary between 65 and 80°C, preferably between 70°C and 75°C. The starting materials placed in the turbo emulsifier are heated to a temperature which may vary from 60 to 80°C, preferably between 65°C and 75°C.

The emulsion is then formed by combining the previously melted starting materials placed in the blender with the previously heated starting materials present in the turbo emulsifier.

The emulsion is then cooled to a temperature which can vary between 45 and 60°C, preferably between 50 and 55°C.

At this point glycerine, Symcalmin (hydroxyphenyl-propamidobenzoic acid, butylene-glycol, pentylene-glycol), the glycolic hippocastanum extract and the preservative can be added to the emulsion. Subsequently, when a temperature which can vary between 30 and 45°C, preferably between 35 and 40°C, the active starting materials Pentapeptide-18 and Acetyl Hexapeptide-8 can be added to the emulsion.

The emulsion is then acidified until it reaches the desired pH.

The composition according to the invention may be used alone or in combination with other active ingredients or methods for treating the symptoms of haemorrhoids.

Another aspect of the invention is represented by a kit comprising the pharmaceutical composition comprising Acetyl Hexapeptide-8 and Pentapeptide-18 alone or in combination with at least one plant extract having activity on the microcirculation and a reusable or disposable applicator containing effective doses of the composition or alternatively there may be pre-dosed single or multi-use packs containing the composition.

The inventors have examined the action of pharmaceutical compositions containing:
- excipients only,
- Pentapeptide-18, Acetyl Hexapeptide-8, plus excipients,
- Pentapeptide-18, Acetyl Hexapeptide-8 and plant extract according to the invention, plus excipients,
- plant extract according to the invention, plus excipients in patients suffering from haemorrhoids.

The inventors have found a surprising improvement in the symptoms of patients treated with the composition containing Pentapeptide-18 and Acetyl Hexapeptide-8 in comparison with that found in patients to whom only the placebo composition was administered. In particular the patients in this treatment group showed a significant reduction in pain and general discomfort due to haemorrhoids.

The inventors then achieved an incredibly high level of improvement in patients using the composition of Pentapeptide-18, Acetyl Hexapeptide-8 and a plant extract according to the invention; in this case, in addition to achieving a reduction in pain and general discomfort, a significant reduction in bleeding was observed. This unexpected improving effect indicates an interaction of the synergistic type and not of the additive type between the components of the composition, which are capable of producing very much more obvious effects than those which would have been expected from a composition containing such ingredients.

The composition according to the invention is therefore capable of bringing out an unexpected improvement in painful conditions of the anal region associated with the symptoms of haemorrhoids, also including the trophism of the haemorrhoidal vascular tissue.

Hitherto the symptomatic topical agents mainly used have been based on cortisones and local anaesthetics which can give rise to sensitisation reactions in the skin and mucosa, above all in the long term; another class of topical agents used, although less frequently, is based on myorelaxants, which however often give rise to headaches and act only on the muscular component, ignoring the vascular component and the symptoms associated with hypersensitivity of the epithelium coating the clusters of haemorrhoidal varices (such as pruritus and discomfort). The new product for topical use benefiting from the synergistic action of its active ingredients makes it possible to improve the symptoms of haemorrhoids by acting on the main physiopathological mechanisms (vascular congestion and sphincter hypertonia) safely, effectively and without side effects.

This therapeutic approach allows patients suffering from early stage haemorrhoids to obtain an improvement in symptoms; patients awaiting surgery can have a bridge-to-surgery treatment which enables them to control symptoms and at the same time "prepare" the tissues for the surgical procedure; in patients who have already undergone surgery it offers an effective coadjuvant treatment during the postoperative period.

The following examples are to be regarded as being illustrative and not limiting the scope of the invention.

### EXAMPLES

### Preparation of the emulsion

The starting materials are placed in the blender and turbo emulsifier in the manner and quantities specified by the formula.

The starting materials placed in the blender melt at a temperature of between 70°C and 75°C. The starting materials placed in the turbo emulsifier should be heated to a temperature of between 65°C and 75°C.

The emulsion is then formed by combining the previously melted starting materials placed in the blender with the previously heated starting materials in the turbo emulsifier.

The emulsion is subsequently cooled until a temperature of between 50 and 55°C is reached.

At this point it is possible to proceed with the addition of glycerine, Symcalmin (hydroxyphenyl-propamidobenzoic acid, butyleneglycol, pentylene-glycol), glycolic hippocastanum extract and the preservative system to the emulsion. Subsequently, on reaching a temperature of between 35 and 40°C, it is possible to proceed with adding the active ingredients Pentapeptide-18 and Acetyl Hexapeptide-8 to the emulsion.

The emulsion is then acidified until the preferred pH is reached.

The stages in the process are illustrated in the diagram below:

### Anonet haemorrhoids flow diagram

Turbo emulsifier
Blender
T=70-75°C
T=65-75°C
Phase B
Phase A
Phase C
Draw phase A into the turbo emulsifier under reduced pressure and stir until an
emulsion is obtained (T1, P1)
Phase D
Stop the heat and stir under medium pressure for 10 minutes (turbo 2, blade 2)
Phase E+F
At T = 50-55°C stir for 3 minutes under medium pressure (turbo 2, blade 2)
Phase G+H
At T = 45-50°C stir for 3 minutes under medium pressure (turbo 2, blade 2)
Phase I
At T = 35-40°C stir for 3 minutes at medium pressure (turbo 2, blade 2)
Phase J
At T = 35-40°C stir for 3 minutes at medium pressure (turbo 2, blade 2)
Phase K
At T = 35-40°C stir for 3 minutes at medium pressure (turbo 2, blade 2)
Phase
L+M
At T = 35-40°C stir for 3 minutes at medium pressure (turbo 2, blade 2)
Phase N
Combine the phases by stirring for 10 minutes under maximum pressure until
bubbles have completely disappeared.
Analysis
Filling
Storage

### COMPONENTS OF THE PHASES:

| ***PHASES*** | ***COMPONENTS*** |
|---|---|
| Phase A | Cetacene (acetylated glycol stearate) |
| | Arlacel 165PAMV (glyceryl stearate PEG-100 stearate) |
| | Acesil 350 (dimethicone) |
| | Xalifin-15 (C12-C20 ACID PEG-8 ester) |
| | Cetylstearyl alcohol 50/50 (cetylstearyl alcohol) |
| | Isoxal-h (polyoxyethylene 10) |
| | Nesatol (C10-C18 triglycerides) |
| | Cutina GMS SE (glyceryl stearate SE) |
| | Symsitive (pentylene F glycol, 4-t-butylcyclohexanol) |
| Phase B | Acqua (aqua) |
| Phase E | Glicerina nat veg fu 99.5% (glycerin) |
| | Protectol PE (phenoxyethanol) |
| Phase G | Potassium sorbate (potassium sorbate) |
| | Preservative G (Imidazolidinyl Urea) |
| | Acqua (aqua) |
| Phase I | Symcalmin (butylene glycol) |
| Phase J | Dermonectin (olisopeptide-5) |
| Phase K | Argirelox (Acetyl Hexapeptide-8-peptide 18) |
| Phase L | Citric Acid (citric acid) |
| | Acqua (aqua) |
| | Hippocastanum extract (Aesculus Hippocastanum extract) |

### Example of a composition containing Acetyl Hexapeptide-8 and Pentapeptide-18 in combination with glycolic hippocastanum extract.

| **INCI nomenclature** | % **weight in formula** |
|---|---|
| AQUA | 52.4220 |
| C10-18 TRIGLYCERIDES | 15.0000 |
| C12-20 ACID/ PEG-8 ESTER | 8.5000 |
| GLYCERINE | 6.0000 |
| GLYCOL-STEARATE ACETYLATE | 2.5000 |
| OLIGOPEPTIDE-5 | 1.9500 |
| PROPYLENE GLYCOL | 1.8000 |
| PENTYLENE GLYCOL | 1.6750 |
| CETYLSTEARYL ALCOHOL | 1.5000 |
| GLYCERYL STEARATE SE | 1.0000 |
| 4-t-BUTYLCYCLOHEXANOL | 0.8000 |
| GLYCERYL STEARATE | 0.7500 |
| PEG-100 STEARATE | 0.7500 |
| PHENOXYETHANOL | 0.6000 |
| DIMETHICONE | 0.5000 |
| BUTYLENE GLYCOL | 0.4750 |
| POTASSIUM SORBATE | 0.4000 |
| ISOCETETH-10 STEARATE | 0.3125 |
| IMIDAZOLIDINYL UREA | 0.3000 |
| CITRIC ACID | 0.2700 |
| ISOSTEARETH-10 STEARATE | 0.1875 |
| AESCULUS HIPPOCASTANUM SEED EXTRACT | 0.0600 |
| CAPRILYL GLYCOL | 0.0500 |
| HYDROXYPHENYL-PROPAMIDOBENZOIC ACID | 0.0500 |
| ACETYL HEXAPEPTIDE-8 | 0.0050 |
| PENTAPEPTIDE-18 | 0.0030 |

### In vivo study

The preliminary study used various compositions to check the efficacy of the various active ingredients.

Patients suffering from haemorrhoids were monitored, each of them completing a form which in addition to identifying the patient showed the instrument examinations, previous treatments, symptoms prior to treatment with the product, checks at different times from the start of application of the product and then the patients' final opinion and any comments on the use of this new substance. A preliminary study was performed to test the differences in efficacy of compositions containing only Acetyl Hexapeptide-8, only Pentapeptide-18 or a mixture of both. The peptides were effective even when alone. It was decided to use a mixture of both for the randomised *in vivo* study.

Patients of both sexes aged between 18 and 70 suffering from haemorrhoids, capable of understanding the proposed study, its purpose and the procedures for implementing it were considered to be eligible for the study. The following were on the contrary excluded: patients suffering from cancer or immunodepression (HIV), patients being treated with anticoagulants, patients suffering from anal abscesses or fistulas, pregnant or lactating women, patients suffering from Crohn's disease, patients suffering from anal incontinence, patients who had previously undergone radiotherapy in the pelvic-perineal region or previous anorectal surgery.

The following were regarded as the main outcomes:
- reduction in anal tone at rest (evaluated using anorectal manometry);
- improvement of symptoms (bleeding, pain, pruritus and discomfort) complained of by patients (using a scale from 0 to 10 for each symptom).

Any adverse events were also recorded.

After clinical evaluation (case history and proctological examination) patients fulfilling the inclusion requirements were selected and these were randomised into 4 different groups:
- **GROUP 1:** placebo cream containing only excipients.
- **GROUP 2:** cream containing the peptides (Acetyl Hexapeptide-8 and Pentapeptide-18) and excipients.
- **GROUP 3:** cream containing only hippocastanum extract and excipients.
- **GROUP 4:** cream containing the peptides (Acetyl Hexapeptide-8 and Pentapeptide-18), hippocastanum extract and excipients.

The creams were prepared as described in the previous examples; in particular the cream provided to group 4 was prepared according to the previous example while for the creams provided to groups 1, 2 and 3 the components which were lacking in individual cases were replaced by water up to volume.

All the patients were asked to apply the cream provided twice a day (1 application every 12 hours) for 30 days. The products were packed in suitable white containers, without labels, which were identical and indistinguishable. The quantity of preparation applied was standardised at 3 grams. In order to avoid confusion among the patients they were provided with a treatment schedule on paper and were instructed how to apply the product both endoanally and to the perianal area.

The patients were assessed at time T0 (time of enrolment), time T1 (after 7 days treatment), and time T2 (after 30 days treatment) by an observer unaware of the treatment assigned. Symptoms complained of were recorded for each patient at each visit: ***bleeding, pain, pruritus and discomfort*** using a *VAS (Visual Analog Scale)*: each patient indicated the severity of symptoms from 0 to 10 on a visual analog scale 10 cm long without interruptions.

The selected patients also underwent anal manometry to evaluate resting sphincter tone at times T0, T1 and T2.

The manometric examination was performed using a silicone catheter of diameter 4.8 mm, provided with eight channels and four radial apertures located 5 cm from the tip and perfused with twice distilled water through a low compliance pump at a rate of 0.75 mL per minute and a flow of 1.2 kg/cm² ((Menfis Biomedica, Bologna, Italy). The catheter was connected to a personal computer through a transponder. The suitably lubricated catheter was introduced into the rectum with the patient in left lateral decubitus. The continuous pull-through technique was used with an automatic retractor set at a rate of 0.5 cm/s.

### Statistical analysis:

The data recorded were placed in a database constructed using Office Excel^{®} software and analysed using Office Excel^{®} and Stata SE14^{®} software.

The variables were expressed as mean ± standard deviation, range or median. The normality of the continuous variables was assessed: those having a normal distribution were compared between the individual times using the Student t test for paired data (parametric method), while variables with a non-normal distribution were compared between the individual times using the Wilcoxon test (non-parametric method). Values of *p* ≤ 0.05 were considered to be significant.

### Results:

10 patients were recruited for each group and no significant differences in sex and age distribution emerged. Comparison between the mean values (VAS scale) recorded for each symptom and the mean values for resting anal pressure at time T0 showed no significant differences between the various groups, which were therefore homogeneous. No adverse events or side effects were recorded in any group of patients and there were no drop-outs.

The data at the various times T0, T1 and T2 were compared for each group.

In the case of the patients in **group 1 (placebo)** no significant differences emerged from the comparison between the means for the VAS values for the various symptoms or the resting anal pressure values at the various times.

The patients in **group 2 (cream containing Acetyl Hexapeptide-8, Pentapeptide-18 and excipients)** showed a statistically significant improvement in pain, discomfort, pruritus and resting anal pressure, but not bleeding.

In the patients in **group 3 (cream containing hippocastanum),** on the other hand, bleeding was reduced without any statistically significant improvement in the other symptoms or basal anal pressures.

The patients in **group 4 (cream containing Acetyl Hexapeptide-8, Pentapeptide-18, 4-t-butylcyclohexanol and hippocastanum)** were the only ones to show statistically significant improvement in all the outcomes assessed - with a significant reduction in bleeding, pain, pruritus and discomfort as well as resting sphincter tone.

### Conclusions:

This preliminary study has shown that the new product for topical use based on a plant extract of **hippocastanum** (with vasotonic, decongesting and antioxidant action), **Acetyl Hexapeptide-8 and Pentapeptide-18** (active in reducing sphincter hypertonia), **4-t-butylcyclohexanol** (with a marked action on pruritus, burning and stinging pain) is safe and effective. Furthermore, as shown by the results, the synergistic effect of the active ingredients demonstrated a better effect than the individual active ingredients, enabling the treated group of patients to achieve a statistically significant improvement in all the outcomes analysed.

## Claims

1. Composition comprising Acetyl Hexapeptide-8 and Pentapeptide-18 in combination with an Hippocastanum plant extract for use in the treatment of haemorrhoids.

2. Composition for use according to claim 1 further comprising at least one plant extract selected from the group of: Centella, Ilex, Vaccinium, Hamamelis, Vitis, Melilotus, Holly, Cypress, Achillea, Ginkgo biloba, Boraginaceae, Urtica, Orthosiphon, Sambucus, Citrus and their mixtures.

3. Composition for use according to claim 1 or 2 in which the Acetyl Hexapeptide-8 is Argireline^{®} and the Pentapeptide-18 is Leuphasyl^{®} and a mixture of Acetyl Hexapeptide-8 and Pentapeptide-18 is Argirelox^{®}.

4. Composition for use according to any one of the previous claims in which the plant extract is a glycolic hippocastanum extract, preferably in concentrations from 0.01% to 10%.

5. Composition for use according to any one of the previous claims further comprising at least one of pharmaceutically acceptable excipients, additives, preservatives, antioxidants, acidifiers, chelating agents, perfumes, rheological additives, solubilising agents, neutralising agents, pharmaceutically acceptable vehicles and their mixtures.

6. Composition for use according to any one of the previous claims further comprising at least one of Imidazolidinyl Urea, potassium sorbate, phenoxyethanol, oligopeptide 5, Dermonectin, glycerine, butyleneglycol, pentylene glycol, hydroxyphenyl-propamidobenzoic acid, 4-t-butylcyclohexanol, polyoxyethylene stearate, glyceryl-stearate, dimethicone, phenyl-trimethicone, pantenol, synthetic or natural triglycerides, fatty acid esters, glycol-stearate or its esters, cetylstearyl alcohol, pantenol, bisabolol, aloe vera, elastin, collagen and their mixtures.

7. Composition for use according to any one of the previous claims in which the Acetyl Hexapeptide-8 is present in a concentration varying between 0.00005% and 0.01%, preferably between 0.003% and 0.007%.

8. Composition for use according to any one of the previous claims in which the Pentapeptide-18 is present in a concentration varying between 0.000025% and 0.005%, preferably between 0.001% and 0.004%.

9. Composition for use according to any of claims 6 to 8 in which Dermonectin is present in a concentration varying between 0.01% and 10%, and preferably between 2% and 6%, or oligopeptide-5 in a concentration varying between 0.004% and 4% and preferably between 0.8% and 2.5%.

10. Composition for use according to any one of claims 6 to 9 in which 4-tbutylcyclohexanol is present in concentrations varying between 0.01% and 3%, preferably between 0.2% and 1.0%.

11. Composition for use according to any one of the previous claims in which the pH varies between 3.5 and 6.5, preferably between 4.5 and 5.5.

12. Composition for use according to any one of the previous claims in which the said composition is in the form of a gel, cream, emulsion, ointment, stick, spray, enema or suppository.

13. Composition for use according to any one of the previous claims for topical administration.

14. Composition for use according to any one of the previous claims in association with at least one active ingredient known for the treatment of haemorrhoids.

15. Kit for use in the treatment of haemorrhoids comprising the composition according to any one of claims 1 to 14 in predetermined dosage units, a reusable or disposable applicator containing effective doses of composition and instructions for use.

## Patentansprüche

1. Zusammensetzung, umfassend Acetyl Hexapeptide-8 und Pentapeptide-18 in Kombination mit einem Pflanzenextrakt von Hippocastanum zur Verwendung in der Behandlung von Hämorrhoiden.

2. Zusammensetzung zur Verwendung nach Anspruch 1, ferner umfassend mindestens einen Pflanzenextrakt, ausgewählt aus der Gruppe Centella, Ilex, Vaccinium, Hamamelis, Vitis, Melilotus, Stechpalme, Zypresse, Achillea, Ginkgo biloba, Boraginaceae, Urtica, Orthosiphon, Sambucus, Citrus und ihre Mischungen.

3. Zusammensetzung zur Verwendung nach Anspruch 1 oder 2, wobei Acetylhexapeptid-8 Argireline^{®} ist und Pentapeptid-18 Leuphasyl^{®} ist und eine Mischung aus Acetylhexapeptid-8 und Pentapeptid-18 Argirelox^{®} ist.

4. Zusammensetzung zur Verwendung nach einem der vorhergehenden Ansprüche, wobei der Pflanzenextrakt ein glykolischer Hippocastanum-Extrakt ist, vorzugsweise in Konzentrationen von 0,01 % bis 10 %.

5. Zusammensetzung zur Verwendung nach einem der vorangehenden Ansprüche, weiterhin umfassend mindestens einen der pharmazeutisch verträglichen Hilfstoffe, Zusatzstoffe, Konservierungsmittel, Antioxidantien, Säuerungsmittel, Chelatbildner, Duftstoffe, rheologischen Zusatzstoffe, Lösungsvermittler, Neutralisationsmittel, pharmazeutisch verträgliche Mittel und deren Mischungen.

6. Zusammensetzung zur Verwendung nach einem der vorhergehenden Ansprüche, die außerdem mindestens eines von Imidazolidinylharnstoff, Kaliumsorbat, Phenoxyethanol, Oligopeptid 5, Dermonectin, Glycerin, Butylenglykol, Pentylenglykol, Hydroxyphenylpropamidobenzoesäure, 4-t-Butylcyclohexanol und Polyoxyethylenstearat, Glycerylstearat, Dimethicon, Phenyltrimethicon, Pantenol, synthetische oder natürliche Triglyceride, Fettsäureester, Glykolstearat oder seine Ester, Cetylstearylalkohol, Pantenol, Bisabolol, Aloe Vera, Elastin, Kollagen und deren Mischungen, umfasst.

7. Zusammensetzung zur Verwendung nach einem der vorhergehenden Ansprüche, wobei Acetylhexapeptid-8 in einer Konzentration zwischen 0,00005 % und 0,01 %, vorzugsweise zwischen 0,003 % und 0,007 % vorliegt.

8. Zusammensetzung zur Verwendung nach einem der vorhergehenden Ansprüche, wobei Pentapeptid-18 in einer Konzentration zwischen 0,000025 % und 0,005 %, vorzugsweise zwischen 0,001 % und 0,004 % vorliegt.

9. Zusammensetzung zur Verwendung nach einem der Ansprüche 6 bis 8, wobei Dermonectin in einer Konzentration zwischen 0,01 % und 10 % und vorzugsweise zwischen 2 % und 6 % oder Oligopeptid-5 in einer Konzentration zwischen 0,004 % und 4 % und vorzugsweise zwischen 0,8 % und 2,5 % vorliegt.

10. Zusammensetzung zur Verwendung nach einem der Ansprüche 6 bis 9, wobei 4-t-Butylcyclohexanol in Konzentrationen zwischen 0,01 % und 3 %, vorzugsweise zwischen 0,2 % und 1,0 % vorliegt.

11. Zusammensetzung zur Verwendung nach einem der vorhergehenden Ansprüche, wobei der pH-Wert zwischen 3,5 und 6,5, vorzugsweise zwischen 4,5 und 5,5, variiert.

12. Zusammensetzung zur Verwendung nach einem der vorhergehenden Ansprüche, wobei die Zusammensetzung in Form eines Gels, einer Creme, einer Emulsion, einer Salbe, eines Stifts, eines Sprays, eines Einlaufs oder eines Zäpfchens vorliegt.

13. Zusammensetzung zur Verwendung nach einem der vorherigen Ansprüche zur topischen Verabreichung.

14. Zusammensetzung zur Verwendung nach einem der vorhergehenden Ansprüche in Verbindung mit mindestens einem Wirkstoff, der zur Behandlung von Hämorrhoiden bekannt ist.

15. Kit zur Verwendung in der Behandlung von Hämorrhoiden, umfassend die Komposition nach einem der Ansprüche 1 bis 14, in vorgegebenen Dosierungseinheiten, der einen wiederverwendbaren oder wegwerfbaren Applikator, wirksame Dosierungen der Zusammensetzung und Verbrauchanweisungen enthält.

## Revendications

1. Composition comprenant Acétyl Hexapeptide-8 et Pentapeptide-18 en combinaison avec un extrait végétal d'hippocastane pour le traitement des hémorroïdes.

2. Composition pour l'utilisation selon la revendication 1, comprenant en outre et au moins un extrait végétal choisi dans le groupe ayant une activité sur la microcirculation, de préférence choisi dans le groupe: Centella, Ilex, Vaccinium, Hamamelis, Vitis, Melilotus, Holly, Cypress, Achillea, Ginkgo biloba, Boraginacées, Urtica, Orthosiphon, Sambucus, Citrus et leurs mélanges.

3. Composition pour l'utilisation selon la revendication 1 ou 2, dans laquelle l'Acétyl Hexapeptide-8 est Argireline^{®} et le Pentapeptide-18 est Leuphasyl^{®} et un mélange d'Acétyl Hexapeptide-8 et de Pentapeptide-18 est Argirelox^{®}.

4. Composition pour l'utilisation selon l'une quelconque des revendications précédentes, dans laquelle l'extrait végétal est un extrait glycolique d'hippocastane, de préférence à des concentrations de 0,01% à 10%.

5. Composition pour l'utilisation selon l'une quelconque des revendications précédentes, comprenant en outre au moins l'un des excipients pharmaceutiquement acceptables, additifs, conservateurs, antioxydants, acidifiants, agents chélatants, parfums, additifs rhéologiques, agents solubilisants, agents neutralisants, véhicules pharmaceutiquement acceptables et leurs mélanges.

6. Composition pour l'utilisation selon l'une quelconque des revendications précédentes, comprenant en outre au moins l'un parmi imidazolidinylurée, sorbate de potassium, phénoxyéthanol, oligopeptide 5, dermonectine, glycérine, butylèneglycol, pentylèneglycol, acide hydroxyphényl-propamidobenzoïque, 4-t-butylcyclohexanol, stéarate de polyoxyéthylène, stéarate de glycéryle, diméthicone, phényl-triméthicone, panténol, triglycérides synthétiques ou naturels, esters d'acides gras, stéarate de glycol ou ses esters, alcool cétylstéarylique, panténol, bisabolol, aloe vera, élastine, collagène et leurs mélanges.

7. Composition pour l'utilisation selon l'une quelconque des revendications précédentes, dans laquelle Acétyl Hexapeptide-8 est présent à une concentration variant entre 0,00005% et 0,01%, de préférence entre 0,003% et 0,007%.

8. Composition pour l'utilisation selon l'une quelconque des revendications précédentes, dans laquelle Pentapeptide-18 est présent à une concentration variant entre 0,000025% et 0,005%, de préférence entre 0,001 % et 0,004%.

9. Composition pour l'utilisation selon l'une quelconque des revendications 6 à 8, dans laquelle Dermonectine est présente à une concentration variant entre 0,01% et 10%, et de préférence entre 2% et 6%, ou oligopeptide-5 à une concentration variant entre 0,004% et 4% et de préférence entre 0,8% et 2,5%.

10. Composition pour l'utilisation selon l'une quelconque des revendications 6 à 9, dans laquelle 4-t-butylcyclohexanol est présent à des concentrations variant entre 0,01 % et 3 %, de préférence entre 0,2 % et 1,0 %.

11. Composition pour l'utilisation selon l'une quelconque des revendications précédentes, dans laquelle le pH varie entre 3,5 et 6,5, de préférence entre 4,5 et 5,5.

12. Composition pour l'utilisation selon l'une quelconque des revendications précédentes, dans laquelle ladite composition se présente sous forme de gel, crème, émulsion, pommade, stick, spray, lavement ou suppositoire.

13. Composition pour l'utilisation selon l'une quelconque des revendications précédentes, pour l'administration topique.

14. Composition pour l'utilisation selon l'une quelconque des revendications précédentes, en association avec au moins un principe actif connu pour le traitement des hémorroïdes.

15. Kit pour utilisation dans le traitement des hémorroïdes comprenant la composition selon l'une quelconque des revendications 1 à 14 en unités posologiques prédéterminées, un applicateur réutilisable ou jetable contenant des doses efficaces de composition et des instructions d'emploi.
